(19) Europäisches Patentamt
European Patent Office
Office européen des brevets

(11) EP 0 718 407 B1

(12) **FASCICULE DE BREVET EUROPEEN**

(45) Date de publication et mention de la délivrance du brevet:
**13.02.2002 Bulletin 2002/07**

(51) Int Cl.[7]: **C12P 41/00**, C07B 57/00, C07C 69/40, C07D 211/18

(21) Numéro de dépôt: **95402839.5**

(22) Date de dépôt: **18.12.1995**

(54) **Intermédiaires de synthèse des énantiomères de l'éliprodil et leur procédé de préparation**

Intermediate für die Synthese von Eliprodilenantiomeren und Verfahren zu ihrer Herstellung

Intermediates for the synthesis of eliprodil enantiomers and process for their preparation

(84) Etats contractants désignés:
**AT BE CH DE DK ES FR GB GR IE IT LI LU NL PT SE**

(30) Priorité: **23.12.1994 FR 9415548**

(43) Date de publication de la demande:
**26.06.1996 Bulletin 1996/26**

(73) Titulaire: **SANOFI-SYNTHELABO
75013 Paris (FR)**

(72) Inventeurs:
- **Zard, Lydia
F-91190 Gif sur Yvette (FR)**
- **Tixidre, Arlette
F-91400 Orsay (FR)**

(74) Mandataire: **Thouret-Lemaitre, Elisabeth et al
Sanofi-Synthélabo Service Brevets 174, avenue de France
75013 Paris (FR)**

(56) Documents cités:
**EP-A- 0 493 617**
**WO-A-92/14830**
**FR-A- 2 696 741**
**US-A- 5 312 950**

- **AGRIC. BIOL. CHEM., vol. 50, no. 9, 1986 pages 2369-2373, H. KUTSUKI ET AL 'Asymmetric hydrolysis of (dl)-1-acyloxy-2-halo-1-phenylethanes with lipase'**
- **JOURNAL OF THE CHEMICAL SOCIETY, 1949 LETCHWORTH GB, pages 203-207, D. WOODCOCK 'Insecticidal activity and chemical constitution. Part I. Chlorinated p-chloroethylbenzene'**
- **TETRAHEDRON: ASYMMETRY, vol. 2, no. 2, 1991 OXFORD GB, pages 113-122, T. R. NIEDUZAK, A. L. MARGOLIN 'Multigram lipase-catalyzed enantioselective acylation in the synthesis of the four stereoisomers of a new biologically active .alpha.-aryl-4-piperidinemethanol derivative'**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 718 407 B1

Printed by Jouve, 75001 PARIS (FR)

## Description

La présente invention a pour objet les énantiomères du 1-(4-chlorophényl)-2-chloroéthanol, leur procédé de préparation et leur utilisation pour la préparation des énantiomères de l'éliprodil et de leurs sels.

L'éliprodil ou α-(4-chlorophényl)-4-[(4-fluorophényl)méthyl] pipéridine-1-éthanol, de formule

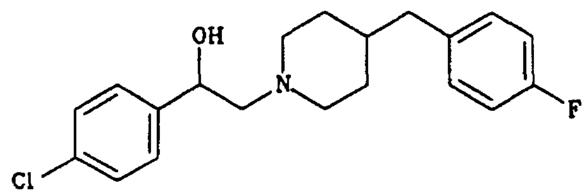

est un composé connu, décrit ainsi que ses propriétés neuro-protectrices, dans le brevet européen n° 0109317.

Un procédé de préparation de ses énantiomères est décrit dans le certificat d'utilité FR 89 04835.

La présente invention concerne un procédé enzymatique de préparation des énantiomères du 1-(4-chlorophényl)-2-chloroéthanol et son application à la préparation des énantiomères de l'éliprodil.

Ce procédé comprend l'hydrolyse enzymatique énantiosélective de l'acétate de (±)-α-(4-chlorophényl)chloroéthyle conduisant à un mélange d'acétate de R(-)-α-(4-chlorophényl)chloroéthyle non hydrolysé et de S(+)-1-(4-chlorophényl)-2-chloroéthanol puis la séparation des constituants du mélange, éventuellement après transformation du S (+)-1-(4-chlorophényl)-2-chloroéthanol en succinate de S(+)-α-(4-chlorophényl)chloroéthyle, et l'hydrolyse de l'acétate de R(-)-α-(4-chlorophényl)chloroéthyle et éventuellement du succinate de S(+)-α-(4-chlorophényl)chloroéthyle.

Selon l'invention, l'hydrolyse de l'acétate de (±)-α-(4-chlorophényl)chloroéthyle est réalisée en milieu aqueux tamponné, par exemple une solution tampon phosphate contenant un mélange de diphosphate de potassium et de phosphate de disodium ou en milieu biphasique constitué d'une solution tampon et d'un solvant organique, tel que toluène, hexane ou t-butylméthyléther.

L'enzyme utilisée pour l'hydrolyse sélective de l'acétate de (±)-α-(4-chlorophényl)chloroéthyle en S(+)-1-(4-chloro phényl)-2-chloroéthanol, est choisie parmi la poudre acétonique de foie de cheval (commercialisée par Sigma), la lipase PS de *Pseudomonas fluorescens* (commercialisée par Amano), la lipase AK de *Pseudomonas* (commercialisée par Amano) et la lipase de *Candida antarctica* sur support ou non (Novozym® SP 435 et SP 525 respectivement, commercialisés par Novo).

L'enzyme, sous forme libre ou bien immobilisée sur support, est utilisée à une concentration de 3 à 50 % en poids par rapport au poids d'acétate de (±)-α-(4-chlorophényl)chloroéthyle.

Les constituants du mélange d'acétate de R(-)-α-(4-chlorophényl)chloroéthyle et de S(+)-1-(4-chlorophényl)-2-chloroéthanol peuvent être séparés par chromatographie.

Le mélange d'acétate de R(-)-α-(4-chlorophényl)chloroéthyle et de S(+)-1-(4-chlorophényl)-2-chloroéthanol peut également être traité par l'anhydride succinique pour donner un mélange d'acétate de R(-)-α-(4-chlorophényl) chloroéthyle et de succinate de S(+)-α-(4-chlorophényl)chloroéthyle. Après traitement par une base, telle que l'hydroxyde de sodium, le succinate de S(+)-α-(4-chlorophényl)chloroéthyle, sous forme de sel, est extrait en phase aqueuse et l'acétate de R(-)-α-(4-chlorophényl)chloroéthyle est extrait en phase organique. Par acidification du milieu contenant le sel de succinate de S(+)-α-(4-chlorophényl)chloroéthyle et extraction en phase organique on obtient le succinate de S(+)-α-(4-chlorophényl) chloroéthyle.

Le R(-)-1-(4-chlorophényl)-2-chloroéthanol et le S(+)-1-(4-chlorophényl)-2-chloroéthanol sont ensuite obtenus respectivement par hydrolyse de l'acétate de R(-)-α-(4-chlorophényl) chloroéthyle et du succinate de S(+)-(4-chlorophényl) chloroéthyle.

Les énantiomères de l'éliprodil sont obtenus par réaction des énantiomères du 1-(4-chlorophényl)-2-chloroéthanol avec la 4-[(4-fluorophényl)méthyl]pipéridine.

Ils peuvent ensuite être transformés en sels d'addition d'acides par traitement par des acides minéraux ou organiques, selon des procédés connus en soi.

L'acétate de (±)-α-(4-chlorophényl)chloroéthyle utilisé comme matière première dans le procédé de l'invention, est préparé par réduction de la 2-chloro-1-(4-chlorophényl) éthanone et acétylation du (±)-1-(4-chlorophényl)-2-chloroéthanol ainsi obtenu, selon le schéma représenté en annexe 1.

Le schéma de synthèse des énantiomères du 1-(4-chlorophényl)-2-chloroéthanol à partir de l'acétate de (±)-α-(4-chlorophényl)chloroéthyle selon le procédé de l'invention, est représenté en annexe 2.

Le schéma de synthèse des énantiomères de l'éliprodil et de leurs chlorhydrates à partir des énantiomères du 1-(4-chloro phényl)-2-chloroéthanol, est représenté en annexe 3.

L'hydrolyse énantiosélective de l'acétate de (±)-1-phényl-2-chloroéthyle est décrite dans Agric. Biol. Chem., 50 (9), 2369-2373, 1986. Par ailleurs, le R (-)-1-(4-chlorophényl)-2-chloroéthanol est décrit dans la demande de brevet EP-0 493 617.

Les exemples suivants illustrent la présente invention, les exemples 1 à 7 concernant la préparation des énantiomères du 1-(4-chlorophényl)-2-chloroéthanol ou de leurs intermédiaires de synthèse selon le procédé de l'invention et les exemples 8 à 12, l'application à la préparation des énantiomères de l'éliprodil et de leurs chlorhydrates.

Exemple 1 : acétate de R(-)-α-(4-chlorophényl)chloroéthyle et S(+)-1-(4-chlorophényl)-2-chloroéthanol

1.1 Acétate de (±)-α-(4-chlorophényl)chloroéthyle

On refroidit par un bain de glace une solution de 100 g (0,54 mol) de 1-(4-chlorophényl)-2-chloroéthanone dans 600 ml de méthanol, puis on ajoute lentement, sous argon et sous agitation, 7,6 g (0,2 mol) de borohydrure de sodium, en maintenant la température du milieu réactionnel inférieure à 15°C. On agite ensuite le mélange pendant 2 h à température ambiante, puis on évapore le méthanol, on verse le résidu dans de l'eau glacée que l'on acidifie par une solution aqueuse d'acide chlorhydrique à 10 % et on extrait avec 3 fois 300 ml de dichlorométhane. On réunit les phases organiques, on les sèche sur sulfate de magnésium et on évapore le solvant sous vide. On reprend le résidu dans 400 ml de dichlorométhane et 43 ml de pyridine, on refroidit la solution par un bain de glace et on ajoute, goutte à goutte, 42 ml (0,59 mol) de chlorure d'acétyle. On chauffe ensuite au reflux pendant 2 h, puis on filtre le mélange réactionnel et on lave le filtrat à l'eau. On sèche la phase organique, on la filtre et on évapore le solvant sous vide. On obtient 116 g de produit sous forme d'huile.

1.2 Acétate de R(-)-α-(4-chlorophényl)chloroéthyle et S(+)-1-(4-chlorophényl)-2-chloroéthanol

On met en suspension 40 g d'acétate de (±)-α-(4-chloro phényl)chloroéthyle dans 500 ml d'une solution tampon phosphate 0,01 M, constituée de biphosphate de potassium et de phosphate de disodium, à 21°C, puis on ajuste le pH à 7,2 par addition d'une solution aqueuse d'acide chlorhydrique et on ajoute 4 g de lipase AK. On laisse réagir pendant 76 h, en maintenant le pH constant par addition d'une solution aqueuse 1 M d'hydroxyde de sodium, puis on extrait la phase aqueuse avec 4 fois 300 ml de dichlorométhane. On réunit les phases organiques, on les sèche sur sulfate de magnésium, on les filtre et on évapore le solvant sous vide. On obtient 34 g d'un produit huileux constitué d'un mélange d'acétate de R(-)-α-(4-chlorophényl)chloroéthyle et de S(+)-1-(4-chlorophényl)-2-chloroéthanol. Par chromatographie sur colonne de silice avec un mélange 95/5 de cyclohexane et d'acétate d'éthyle, on obtient :
13,85 g de S(+)-1-(4-chlorophényl)-2-chloroéthanol
$[\alpha]_D^{20}$ = + 43,8° (c = 0,974 ; chloroforme)
ee : 100 % [HPLC chirale : phénylglycine covalente (Pirkle),
230 nm, 0,6 ml/mn, hexane/isopropanol (99/1)], et
15 g d'acétate de R(-)-α-(4-chlorophényl)chloroéthyle
$[\alpha]_D^{20}$ = - 64,7° (c = 1,17 ; chloroforme)
ee > 98 % (HPLC chirale, mêmes conditions que précédemment).

Exemple 2 : acétate de R(-)-α-(4-chlorophényl)chloroéthyle et S(+)-1-(4-chlorophényl)-2-chloroéthanol

Selon le procédé de l'étape 2 de l'exemple 1, à partir de 20 g d'acétate de (±)-α-(4-chlorophényl)chloroéthyle, 350 ml de tampon phosphate et 6 g de lipase PS de *Pseudomonas fluorescens,* que l'on fait réagir pendant 42 h, on obtient après chromatographie, 9 g d'acétate de R(-)-α-(4-chlorophényl)chloroéthyle (ee : 99 %) et 6,95 g de S(+)-1-(4-chlorophényl)-2-chloroéthanol (ee > 98 %).

Exemple 3 : acétate de R(-)-α-(4-chlorophényl)chloroéthyle et S(+)-1-(4-chloronhényl)-2-chloroéthanol

Selon le procédé de l'étape 2 de l'exemple 1, on traite 20 mg d'acétate de (±)-α-(4-chlorophényl)chloroéthyle dans 0,5 ml de tampon phosphate, par 2 mg de Novozym® SP 525, et on suit l'évolution de la réaction par HPLC chirale. Au bout de 24 h, on trouve : ee > 97 % pour l'acétate de R(-)-α-(4-chloro phényl)chloroéthyle et pour le S(+)-1-(4-chloro phényl)-2-chloroéthanol.

Exemple 4 : acétate de R(-)-α-(4-chlorophényl)chloroéthyle et S(+)-1-(4-chlorophényl)-2-chloroéthanol

Selon le procédé de l'étape 2 de l'exemple 1, on traite 6 g d'acétate de (±)-α-(4-chlorophényl)chloroéthyle dans 75 ml de tampon phosphate, par 600 mg de Novozym® SP 435, et on suit l'évolution de la réaction par HPLC

chirale. Au bout de 3 jours, on dilue le milieu réactionnel avec 50 ml de dichlorométhane puis on le filtre. On extrait ensuite la phase aqueuse avec 3 fois 50 ml de dichlorométhane, on réunit les phases organiques, on les sèche et on les concentre sous pression réduite pour obtenir 5,34 g de produit sous forme d'huile. On reprend le résidu dans un peu de cyclohexane, puis on le chromatographie sur colonne de silice d'abord avec un mélange 99/1, puis avec un mélange 90/10 de cyclohexane et d'acétate d'éthyle. On obtient 2,66 g d'acétate de R(-)-α-(4-chlorophényl)chloroéthyle (ee = 86 %) et 2,06 g de S(+)-1-(4-chlorophényl)-2-chloroéthanol (ee = 97 %).

Exemple 5 : acétate de R(-)-α-(4-chlorophényl)chloroéthyle et succinate de S(+)-α-(4-chlorophényl)chloroéthyle

On introduit dans un réacteur de 50 l, 40 l d'eau déminéralisée, 44,5 g de dihydrate d'hydrogénophosphate disodique, 20,4 g de dihydrogénophosphate de potassium et 2382 g d'acétate de (±)-α-(4-chlorophényl)chloroéthyle et on ajuste le pH à 7,2 par addition d'une solution 1 M d'hydroxyde de sodium. On ajoute ensuite, sous agitation, 92,7 g de lipase SP 525 et on injecte une solution 1 M d'hydroxyde de sodium de façon à maintenir le pH à 7,0 ± 0,2.
Au bout de 48 heures, après addition de 4,5 l d'hydroxyde de sodium, on extrait la suspension par un mélange de 25 l de toluène et 35 l d'éthanol, puis par un mélange de 25 l de toluène et 15 l d'éthanol. On réunit les phases organiques supérieures, on les évapore à sec, on reprend le résidu par 10 l de t-butylméthyléther et 3 kg de sulfate de sodium, puis on filtre, on rince le sulfate avec 5 l de t-butylméthyléther et on évapore la solution éthérée à sec. On obtient ainsi 1927 g d'un mélange sous forme d'huile contenant l'acétate de R(-)-α-(4-chlorophényl)chloroéthyle (ee = 99,06 %) et le S(+)-1-(4-chlorophényl)-2-chloroéthanol (ee = 86,0 %).
On dissout ce mélange dans 11 l de toluène puis on ajoute 641 g de triéthylamine et 634 g d'anhydride succinique. On laisse le mélange sous agitation à température ambiante pendant une nuit puis on extrait les composés acides par une solution de 530 ml d'hydroxyde de sodium à 30 % dans 6 l d'eau déminéralisée. Après décantation, on réextrait la phase toluénique avec 3 l d'eau déminéralisée puis on réunit les phases aqueuses et on les lave avec 3 l de toluène. On réunit les phases toluèniques, on les évapore à sec puis on dissout les 1189 g de produit obtenu dans de l'hexane. Après absorption sur 1,75 kg de silice, élution avec 1 l d'hexane, 4 l d'un mélange 90/10, 3 l d'un mélange 80/20 et 1 l d'un mélange 50/50 d'hexane et d'acétate d'éthyle et évaporation du solvant, on obtient 1056 g d'acétate de R(-)-α-(4-chlorophényl)chloroéthyle.
On réunit les phases aqueuses basiques puis on les acidifie par addition d'acide chlorhydrique à 36 % jusqu'à pH = 5. On extrait ensuite le succinate acide avec 2 fois 2 l de dichlorométhane, on évapore le solvant et on redissout les 1405 g de résidu huileux obtenu dans du dichlorométhane. Après absorption sur 2 kg de silice et élution avec 4 l de dichlorométhane, 2 l d'un mélange 90/10 et 2 l d'un mélange 50/50 de dichlorométhane et de méthanol, puis 1 l de méthanol et évaporation du solvant, on obtient 1349 g de succinate de S(+)-α-(4-chlorophényl)chloroéthyle sous forme d'huile.

Exemple 6 : S(+)-1-(4-chlorophényl)-2-chloroéthanol

A une solution de 1340 g de succinate de S(+)-α-(4-chlorophényl)chloroéthyle dans 7,5 l de méthanol refroidie à 0°C, on ajoute en 1 heure, en maintenant la température inférieure à 6°C, 530 g de chlorure d'acétyle. On chauffe ensuite le mélange à reflux pendant 9 heures puis on concentre à sec sous pression réduite. On obtient 1053 g d'un mélange de S(+)-1-(4-chloro phényl)-2-chloroéthanol et de succinate de diméthyle.
$[\alpha]_D^{20}$ du mélange = + 23,6° (c = 1 ; chloroforme)
ee = 86,6 % (HPLC chirale).

Exemple 7 : R(-)-1-(4-chlorophényl)-2-chloroéthanol

On dissout 1050 g d'acétate de R(-)-α-(4-chlorophényl) chloroéthyle dans 7 l de méthanol puis on refroidit le mélange réactionnel dans un bain de glace et on ajoute 460 g de chlorure d'acétyle en 1 heure, en maintenant la température inférieure à 5°C. On chauffe ensuite à 50°C pendant 1 h, puis on concentre à sec. On obtient 820 g de produit.
$[\alpha]_D^{20}$ = - 47,2° (c = 1 ; chloroforme).
ee = 97,7 %.

Exemple 8 : S(+)-α-(4-chlorophényl)-4-[(4-fluorophényl) méthyl]pipéridine-1-éthanol

On dissout 19 g (0,1 mol) de S(+)-1-(4-chlorophényl)-2-chloroéthanol obtenu selon l'un des exemples 1 à 3, dans 230 ml d'isopropanol, puis on ajoute 33 ml d'eau, 16,35 g (0,12 mol) de carbonate de potassium et 22,5 g (0,1 mol) de 4-[(4-fluorophényl)méthyl]pipéridine. On chauffe le mélange réactionnel au reflux pendant 3 h, puis on le laisse revenir à température ambiante, on filtre le précipité formé et on le sèche. On reprend le résidu solide par de l'eau,

puis on filtre le produit insoluble, on le sèche et on le recristallise dans 350 ml d'éthanol absolu au reflux.
On obtient 20,2 g de produit.
Point de fusion : 142-143°C.
$[\alpha]_D^{20}$ = + 47,9° (c = 1,015 ; chloroforme)
ee > 99,8 % (HPLC chirale).

Exemple 9 : S(+)-α-(4-chlorophényl)-4-[(4-fluorophényl) méthyl]pipéridine-1-éthanol

Dans un réacteur de 10 l maintenu sous azote, on introduit 5 l d'isopropanol, 0,93 l d'eau, 851 g de carbonate de potassium, 643 g de chlorhydrate de 4-[(4-fluorophényl) méthyl]pipéridine et 978 g du mélange de S(+)-1-(4-chloro phényl)-2-chloroéthanol et de succinate de diméthyle obtenu selon l'exemple 6. On rajoute 1,4 l d'isopropanol puis on chauffe à reflux pendant 4 heures. On refroidit ensuite la suspension à 15°C, on la filtre puis on rince le solide avec 1 l d'isopropanol, on le met en suspension dans 8 l d'eau et on agite pendant 1 h 15 mn. Après filtration et lavage à l'eau, on sèche le solide pendant 1 nuit à 50°C sous pression réduite. On recristallise le produit obtenu dans 9,8 l d'éthanol.
On obtient 608 g de produit.
Point de fusion : 144-144,3°C.
$[\alpha]_D^{20}$ = + 46,8° (c = 1 ; chloroforme)
ee = 99,4 %.

Exemple 10 : R(-)-α-(4-chlorophényl)-4-[(4-fluorophényl) méthyl]pipéridine-1-éthanol

A partir de 9,1 g de R(-)-1-(4-chlorophényl)2-chloroéthanol et de 10,76 g de 4-[(4-fluorophényl)méthyl]pipéridine, traités dans les conditions de l'exemple 8, on obtient 12,2 g de produit.
Point de fusion : 142-143°C.
$[\alpha]_D^{20}$ = - 47,9° (c = 1,015 ; chloroforme)
ee > 99,8 %.

Exemple 11 : chlorhydrate de R(-)-α-(4-chlorophényl)-4-[(4-fluorophényl)méthyl]pipéridine-1-éthanol

A une suspension de 495 g de R(-)-α-(4-chlorophényl)-4-[(4-fluorophényl)méthyl]pipéridine-1-éthanol dans 1,5 l d'éthanol, on ajoute 130 ml d'acide chlorhydrique concentré et on chauffe jusqu'à dissolution. On refroidit ensuite par un bain de glace, on filtre le précipité formé et on le rince avec 0,25 l d'éthanol. On recristallise dans 2 l d'éthanol les 465 g de produit obtenus. On obtient ainsi 415 g de produit pur.
Point de fusion : 216,8°C.
$[\alpha]_D^{20}$ = - 34° (c = 1 ; méthanol)
ee > 99,9%.

Exemple 12 : chlorhydrate de S(+)-α-(4-chlorophényl)-4-[(4-fluorophényl)méthyl]pipéridine-1-éthanol

Selon le procédé décrit à l'exemple 11, on obtient à partir du S(+)-α-(4-chlorophényl)-4-[(4-fluorophényl)méthyl] pipéridine-1-éthanol, le chlorhydrate de S(+)-α-(4-chloro phényl)-4-[(4-fluorophényl)méthyl]pipéridine-1-éthanol.
Point de fusion : 217,1°C.
$[\alpha]_D^{20}$ = + 34,7° (c = 1 ; méthanol)
ee = 99,4 %.

## Annexe 1

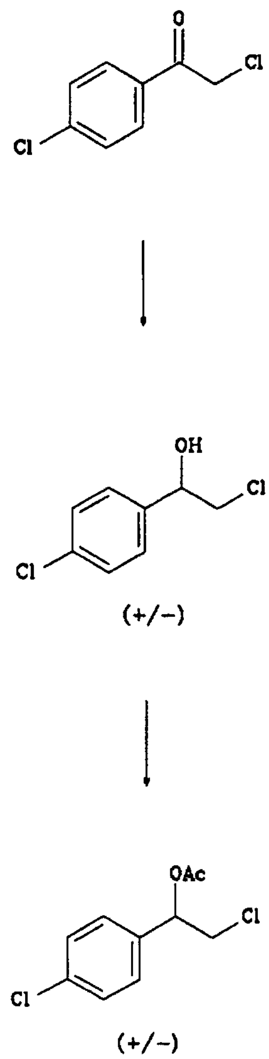

## Annexe 2

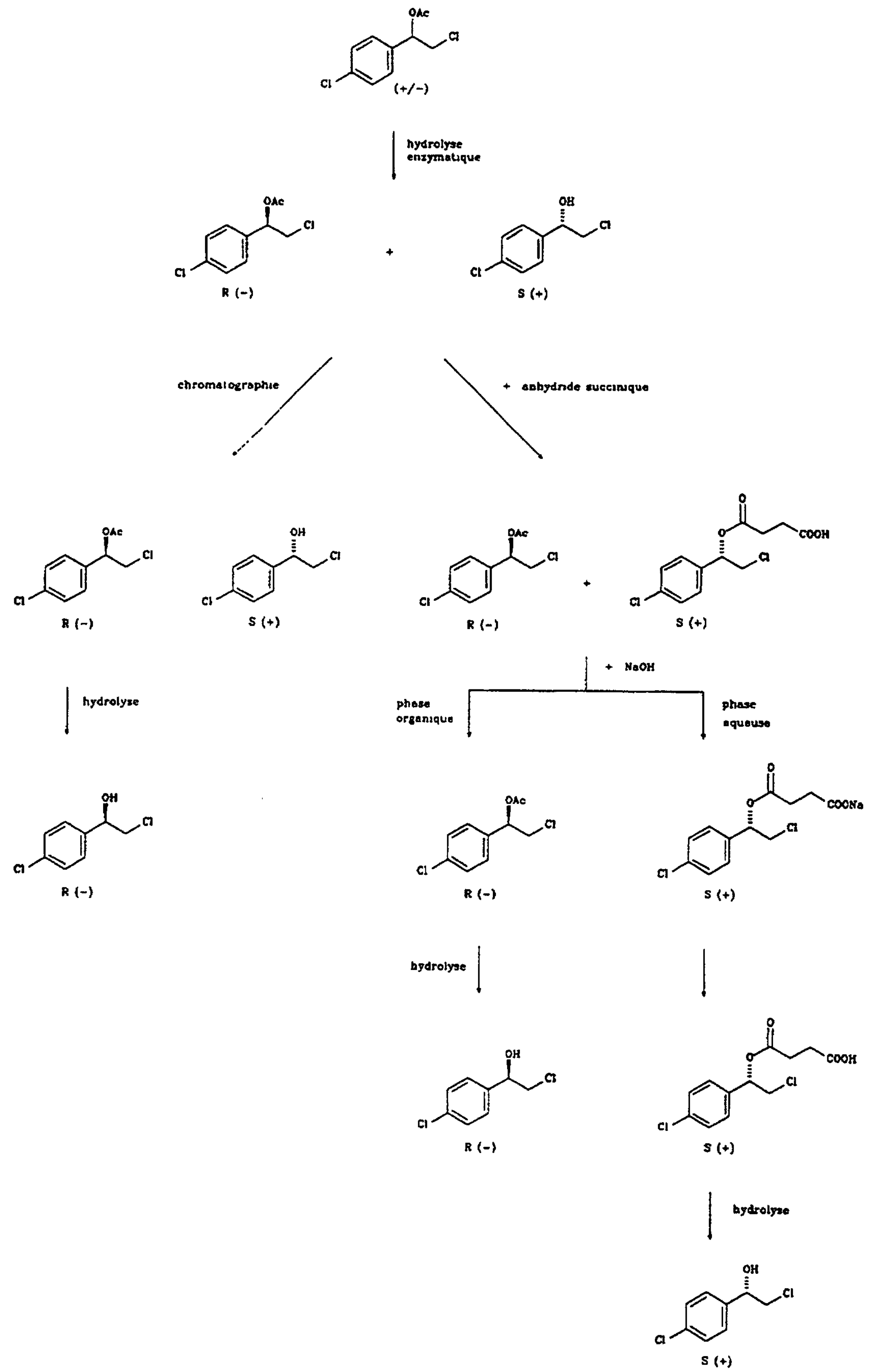
(+/-)
hydrolyse enzymatique
R (-)
+
S (+)
chromatographie
+ anhydride succinique
R (-)
S (+)
R (-)
+
S (+)
hydrolyse
+ NaOH
phase organique
phase aqueuse
R (-)
R (-)
S (+)
hydrolyse
R (-)
S (+)
hydrolyse
S (+)

## Annexe 3

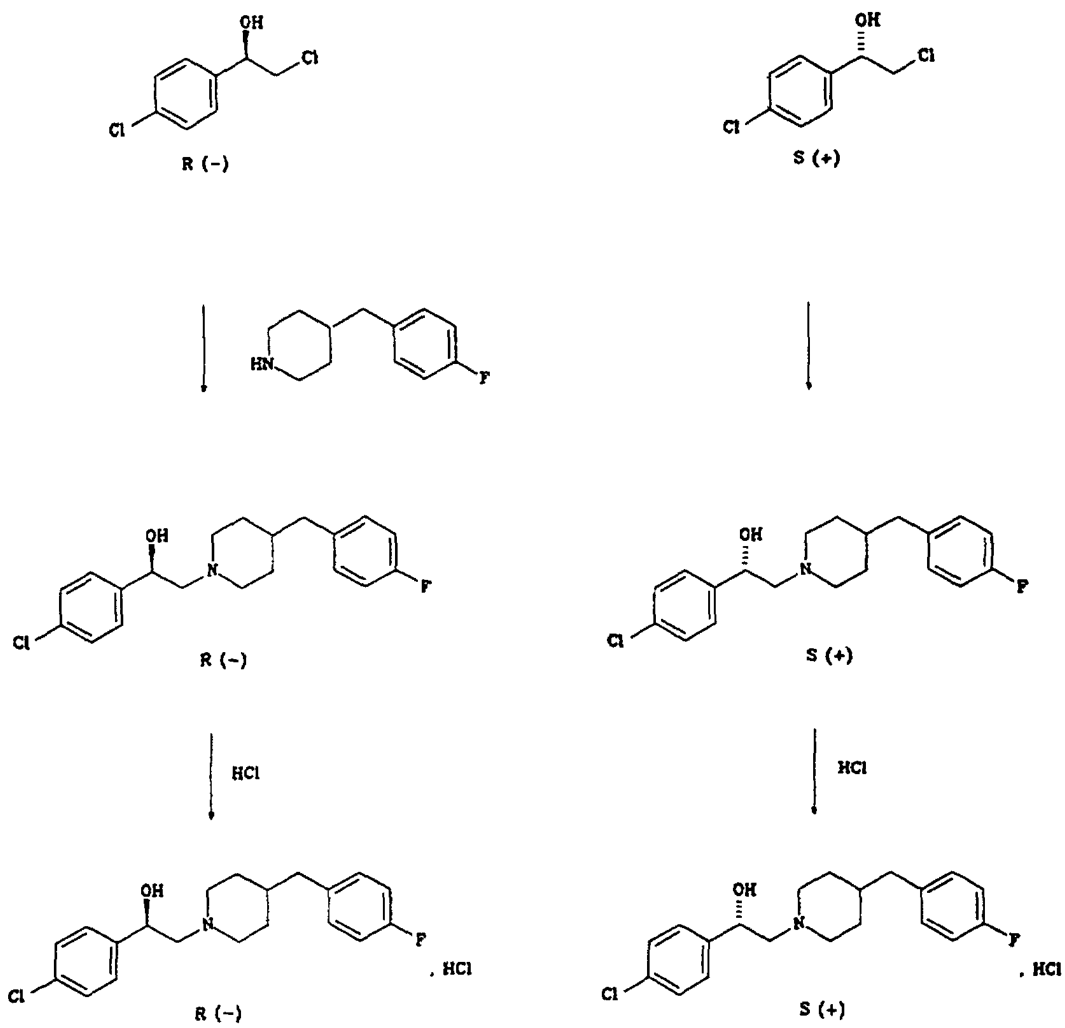

R (-) S (+)

R (-) S (+)

R (-) S (+)

## Revendications

1. Procédé de préparation des énantiomères du 1-(4-chlorophényl)-2-chloroéthanol, **caractérisé en ce qu'**il comprend l'hydrolyse enzymatique énantiosélective de l'acétate de (±)- α-(4-chlorophényl)chloroéthyle au moyen d'une enzyme choisie parmi la poudre acétonique de foie de cheval, la lipase PS de *Pseudomonas fluorescens*, la lipase AK de *Pseudomonas* et la *lipase de Candida antarctica,* la séparation de l'acétate de R(-)-α-(4-chlorophényl)chloroéthyle non hydrolysé et du S(+)-1-(4-chlorophényl)-2-chloroéthanol, éventuellement après sa transformation en succinate de S(+)-α-(4-chlorophényl)chloroéthyle, puis l'hydrolyse de l'acétate de R(-)-α-(4-chlorophényl)chloroéthyle et éventuellement du succinate de S(+)-α-(4-chlorophényl)-chloroéthyle.

2. Procédé selon la revendication 1 **caractérisé en ce qu'**il comprend la séparation chromatographique de l'acétate de R(-)-α-(4-chlorophényl)chloroéthyle et du S(+)-1-(4-chloro phényl)-2-chloroéthanol et l'hydrolyse de l'acétate de R(-)-α-(4-chlorophényl)chloroéthyle.

3. Procédé selon la revendication 1 **caractérisé en ce qu'**il comprend le traitement du mélange d'acétate de R(-)-α-(4-chlorophényl)chloroéthyle et de S(+)-1-(4-chlorophényl)-2-chloroéthanol par l'anhydride succinique pour donner un mélange d'acétate de R(-)-α-(4-chlorophényl)chloroéthyle et de succinate de S(+)-α-(4-chlorophényl)chloroéthyle, la séparation des constituants du mélange par extraction du succinate de S(+)-α-(4-chlorophényl)chloroéthyle sous forme de sel en phase aqueuse et de l'acétate de R(-)-α-(4-chlorophényl)chloroéthyle en phase organique, l'extraction en phase organique du succinate de S(+)-α-(4-chlorophényl) chloroéthyle et l'hydrolyse de l'acétate de R(-)-α-(4-chlorophényl)chloroéthyle et du succinate de S(+)-α-(4-chlorophényl)chloroéthyle.

4. Procédé selon l'une quelconque des revendications 1 à 3, **caractérisé en ce que** l'enzyme est immobilisée sur support.

5. Procédé selon l'une quelconque des revendications 1 à 4, **caractérisé en ce que** la concentration en poids d'enzyme par rapport au poids d'acétate de (±)-α-(4-chlorophényl)chloroéthyle est comprise entre 3 et 50 %.

6. Le S(+)-1-(4-chlorophényl)-2-chloroéthanol obtenu par le procédé selon l'une quelconque des revendications 1 à 5.

7. Application du S(+)-1-(4-chlorophényl)-2-chloroéthanol obtenu par le procédé selon l'une quelconque des revendications 1 à 5, à la préparation du S(+)-α-(4-chlorophényl)-4-[(4-fluorophényl)méthyl]pipéridine-1-éthanol et de ses sels.

8. Application du S(+)-1-(4-chlorophényl)-2-chloroéthanol, selon la revendication 7, à la préparation du chlorhydrate de S(+)-α-(4-chlorophényl)-4-[(4-fluorophényl)méthyl] pipéridine-1-éthanol.

9. Application du R(- )-1-(4-chlorophényl)-2-chloroéthanol obtenu par le procédé selon l'une quelconque des revendications 1 à 5, à la préparation du R(-)-α-(4-chlorophényl)-4-[(4-fluorophényl)méthyl]pipéridine-1-éthanol et de ses sels.

10. Application du R(-)-1-(4-chlorophényl)-2-chloroéthanol, selon la revendication 9, à la préparation du chlorhydrate de R(-)-α-(4-chlorophényl)-4-[(4-fluorophényl)méthyl] pipéridine-1-éthanol.

## Claims

1. Process for the preparation of the enantiomers of 1-(4-ch]orophenyl)-2-chloroethanol, **characterized in that** it comprises the enantioselective enzymatic hydrolysis of (±)-α-(4-chlorophenyl)chloroethyl acetate by means of an enzyme chosen from horse liver acetone powder, lipase PS from *Pseudomonas fluorescens,* lipase AK from *Pseudomonas* and the lipase from *Candida antarctica,* the separation of unhydrolysed R-(-)-α-(4-chlorophenyl)chloroethyl acetate and of S-(+)-1-(4-chlorophenyl)-2-chloroethanol, optionally after its conversion to S-(+)-α-(4-chlorophenyl)chloroethyl succinate, and then the hydrolysis of R-(-)-α-(4-chlorophenyl)chloroethyl acetate and optionally of S-(+)-α-(4-chlorophenyl)chloroethyl succinate.

2. Process according to Claim 1, **characterized in that** it comprises the chromatographic separation of R-(-)-α-(4-chlorophenyl)chloroethyl acetate and of S-(+)-1-(4-chlorophenyl)-2-chloroethanol and the hydrolysis of R-(-)-α-(4-chlorophenyl)chloroethyl acetate.

3. Process according to Claim 1, **characterized in that** it comprises the treatment of the mixture of R-(-)-α-(4-chlorophenyl)chloroethyl acetate and of S-(+)-1-(4-chlorophenyl)-2-chloroethanol with succinic anhydride in order to give a mixture of R-(-)-α-(4-chlorophenyl)chloroethyl acetate and of S-(+)-α-(4-chlorophenyl)chloroethyl succinate, the separation of the constituents of the mixture by extraction of S-(+)-α-(4-chlorophenyl)chloroethyl succinate in the salt form into the aqueous phase and of R-(-)-α-(4-chlorophenyl)chloroethyl acetate into the organic phase, the extraction into the organic phase of S-(+)-α-(4-chlorophenyl)chloroethyl succinate and the hydrolysis of R-(-)-α-(4-chlorophenyl)chloroethyl acetate and of S-(+)-α-(4-chlorophenyl)chloroethyl succinate.

4. Process according to any one of Claims 1 to 3, **characterized in that** the enzyme is immobilized on a support.

5. Process according to any one of Claims 1 to 4, **characterized in that** the concentration by weight of enzyme with respect to the weight of (±)-α-(4-chlorophenyl)chloroethyl acetate is between 3 and 50%.

6. S-(+)-1-(4-Chlorophenyl)-2-chloroethanol obtained by the process according to any one of Claims 1 to 5.

7. Application of S-(+)-1-(4-chlorophenyl)-2-chloroethanol obtained by the process according to any one of Claims 1 to 5 to the preparation of S-(+)-α-(4-chlorophenyl)-4-[(4-fluorophenyl)methyl]piperidine-1-ethanol and of its salts.

8. Application of S-(+)-1-(4-chlorophenyl)-2-chloroethanol according to Claim 7 to the preparation of S-(+)-α-(4-chlorophenyl)-4-[(4-fluorophenyl)methyl]-piperidine-1-ethanol hydrochloride.

9. Application of R-(-)-1-(4-chlorophenyl)-2-chloroethanol obtained by the process according to any one of Claims 1 to 5 to the preparation of R-(-)-α-(4-chlorophenyl)-4-[(4-fluorophenyl)methyl]piperidine-1-ethanol and of its salts.

10. Application of R-(-)-1-(4-chlorophenyl)-2-chloroethanol according to Claim 9 to the preparation of R-(-)-α-(4-chlorophenyl)-4-[(4-fluorophenyl)-methyl]piperidine-1-ethanol hydrochloride.

## Patentansprüche

1. Verfahren zur Herstellung der Enantiomeren von 1-(4-Chlorphenyl)-2-chlorethanol, **dadurch gekennzeichnet, daß** es die enantioselektive enzymatische Hydrolyse von (±)-α-(4-Chlorphenyl)-chlorethylacetat mit Hilfe eines Enzyms ausgewählt aus dem Acetonpulver von Pferdeleber, Lipase PS von *Pseudomonas fluorescens.* Lipase AK von *Pseudomonas* und *Lipase von Candida antarctica*, die Trennung des nicht hydrolysierten R(-)-α-(4-(Chlorphenyl)-chlorethyl-acetats von S(+)-1-(4-Chlorphenyl)-2-chlorethanol, gegebenenfalls nach dessen Umwandlung in S(+)-α-(4-Chlorphenyl)-chlorethyl-succinat und dann die Hydrolyse von R(-)-α-(4-Chlorphenyl)-chlorethyl-acetat und gegebenenfalls von S(+)-α-(4-Chlorphenyl)-chlorethyl-succinat umfaßt.

2. Verfahren nach Anspruch 1, **dadurch gekennzeichnet, daß** es die chromatographische Trennung von R(-)-α-(4-Chlorphenyl)-chlorethyl-acetat und S(+)-1-(4-Chlorphenyl)-2-chlorethanol und die Hydrolyse von R(-)-α-(4-Chlorphenyl)-chlorethyl-acetat umfaßt.

3. Verfahren nach Anspruch 1, **dadurch gekennzeichnet, daß** es die Behandlung der Mischung aus R(-)-α-(4-Chlorphenyl)-chlorethyl-acetat und S(+)-1-(4-Chlorphenyl)-2-chlorethanol mit Bernsteinsäureanhydrid zur Bildung von R(-)-α-(4-Chlorphenyl)-chlorethyl-acetat und S(+)-α-(4-Chlorphenyl)-chlorethyl-succinat, die Trennung der Bestandteile der Mischung durch Extraktion des S(+)-α-(4-Chlorphenyl)-chlorethyl-succinats in Form des Salzes in die wäßrige Phase und des R(-)-α-(4-Chlorphenyl)-chlorethyl-acetats in die organische Phase, die Extraktion des S(+)-α-(4-Chlorphenyl)-chlorethyl-succinats in die organische Phase und die Hydrolyse des R(-)-α-(4-Chlorphenyl)-chlorethyl-acetats und des S(+)-α-(4-Chlorphenyl) -chlorethyl-succinats umfaßt.

4. Verfahren nach einem der Ansprüche 1 bis 3, **dadurch gekennzeichnet, daß** das Enzym auf einem Träger immobilisiert ist.

5. Verfahren nach einem der Ansprüche 1 bis 4, **dadurch gekennzeichnet, daß** die Gewichtskonzentration des Enzyms, bezogen auf das Gewicht des (±)-α-(4-Chlorphenyl)-chlorethyl-acetats zwischen 3 und 50 % liegt.

6. S(+)-1-(4-Chlorphenyl)-2-chlorethanol erhalten nach dem Verfahren nach einem der Ansprüche 1 bis 5.

7. Verwendung des nach dem Verfahren eines der Ansprüche 1 bis 5 erhaltenen S(+)-1-(4-Chlorphenyl)-2-chlorethanols für die Herstellung von S(+)-α-(4-Chlorphenyl) -4-[(4-fluorphenyl)-methyl]-piperidin-1-ethanol und dessen Salzen.

8. Verwendung von S(+)-1-(4-Chlorphenyl)-2-chlorethanol nach Anspruch 7 für die Herstellung des Hydrochlorids von S(+)-α-[4-Chlorphenyl]-4-[(4-fluorphenyl)-methyl]-piperidin-1-ethanol.

9. Verwendung des nach dem Verfahren eines der Ansprüche 1 bis 5 erhaltenen R(-)-1-(4-Chlorphenyl)-2-chlorethanols für die Herstellung von R(-)-α-(4-Chlorphenyl)-4-[(4-fluorphenyl)-methyl]-piperidin-1-ethanol und dessen Salzen.

10. Verwendung von R(-)-1-(4-Chlorphenyl)-2-chlorethanol nach Anspruch 9 für die Hertsellung des Hydrochlorids von R(-)-α-(4-Chlorphenyl)-4-[(4-fluorphenyl)-methyl]-piperidin-1-ethanol.